# EUROPEAN PATENT SPECIFICATION

(11) **EP 0 610 317 B1**
(45) Date of publication and mention of the grant of the patent: **31.01.2001**
(21) Application number: 92922300.6
(22) Date of filing: 21.10.1992
(51) Int. Cl.: C07K 5/06, C07K 5/10, C07K 7/06, C07K 7/08, C07F 5/02, A61K 31/69, A61K 38/04

(54) **INHIBITORS OF DIPEPTIDYL-AMINOPEPTIDASE TYPE IV**
INHIBITOREN DER DIPEPTIDYL-AMINOPEPTIDASE VOM TYP-IV
INHIBITEURS DE DIPEPTIDYL-AMINOPEPTIDASE DE TYPE IV

(30) Priority: 22.10.1991 US 781552
(43) Date of publication of application: 17.08.1994
(60) Divisional application: 00201402.5
(73) Proprietor: NEW ENGLAND MEDICAL CENTER HOSPITALS, INC., Boston Massachusetts 02111 (US); TUFTS UNIVERSITY, Medford, MA 02155 (US)
(72) Inventor: BACHOVCHIN, William, W., Melrose, MA 02176 (US); PLAUT, Andrew, G., Boston, MA 02111 (US); FLENTKE, George, R., Boston, MA 02111 (US)
(74) Representative: Jump, Timothy John Simon
(86) International application number: US9209026
(87) International publication number: WO9308259

(56) References cited:
- WO-A-91/16339
- US-A- 4 318 904
- US-A- 4 582 821
- US-A- 4 636 492
- US-A- 4 652 552
- US-A- 4 935 493
- US-A- 4 999 082
- PROC.NATL.ACAD.SCI. USA, vol. 88, 15 February 1991, pages 1556-1559, XP002011727 FLENTKE G.R. ET AL.: "Inhibition of dipeptidyl aminopeptidase IV (DP-IV) by Xaa-boroPro dipeptides and use of these inhibitors to examine the role of DP-IV in T-cell function"
- THE JOURNAL OF BIOLOGICAL CHEMISTRY, vol. 265, no. 7, 5 March 1990, BALTIMORE, MD, US, pages 3738-3743, XP002011728 BACHOVCHIN W.W. ET AL.: "Inhibition of IgA1 Proteinases from Nesseria gonorrhoeae and Hemophilus influenzae by Peptide Prolyl Boronic Acids"
- BIOCHEMISTRY, vol. 27, 4 October 1988, pages 7682-7688, XP002011729 KETTNER CH. A. ET AL.: "Kinetic Properties of the Binding of alpha-Lytic Protease to Peptide Boronic Acids"

## Description

This invention relates to inhibitors of the amino peptidase activity of dipeptidyl peptidase type IV (DP-IV).

DP-IV is a postproline cleaving enzyme with a specificity for removing Xaa-Pro (where Xaa represents any amino acid) dipeptides from the amino terminus of polypeptides. DP-IV will also remove Xaa-Ala dipeptides from amino termini, albeit less efficiently. DP-IV is present in many mammalian cells and tissues, for example, renal tubule cells, intestinal epithelium, and blood plasma. It is also present on the surface of CD4+ and some CD8+ T-cells. It is thought to be involved in the regulation of the immune response; occurrence of DP-IV on a cell surface is associated with the ability of cells to produce interleukin-2 (IL-2). DP-IV is also referred to as dipeptidyl-peptide hydrolase DAP-IV or DPP-IV; it is assigned EC number 3.4.14.5.

Three different inhibitors of DP-IV are known. One of these is a suicide inhibitor: N-Ala-Pro-O-(nitrobenzyl-) hydroxylamine. (The standard three letter amino acid codes are used in this application; O represents oxygen.) Another is a competitive inhibitor: e-(4-nitro) benzoxycarbonyl-Lys-Pro. The third is a polyclonal rabbit anti-porcine kidney DP-IV immunoglobulin.

Peptides containing aminoboromic acids are known as inhibitor of DP-IV (See Flentke et al. Proc. Natl. Acad. Sci. USA 88 : 1556-1559 (1991) ; Kettner et al. Biochemistry 27 : 7682-7688 (1988)) and of proteinases in general (See US-A-4,935,493 and Bachovchin et al. J. Biol. Chem. 265: 3738-3743 (1990).

The enzymatic activity of DP-IV involves cleaving of a dipeptide from the free amino terminus of a polypeptide. DP-IV has a preference for cleaving after a proline, i.e., a proline in the penultimate position from the amino terminus. A free amino terminus is required; thus, DP-IV is a postproline cleaving enzyme with a specificity for removing an N-terminal Xaa-Pro dipeptide from a polypeptide (where Xaa can be any amino acid, including proline). DP-IV also will remove a Xaa'-Ala dipeptide from an amino terminus of a polypeptide when Xaa' is an amino acid with a bulky side group, e.g., tyrosine.

This invention concerns provision of potent inhibitors of the enzymatic activity of DP-IV. Generally, an α-amino boronic acid analog of proline (boroPro is used to designate one such analog which has the carboxyl group of proline replaced with a B(OH)₂ group, where (OH)₂ represents two hydroxyl groups and B represents boron) is bonded to an amino acid to form a dipeptide with boroPro as the carboxy terminal residue. These dipeptide prolyl-boronic acids are potent and highly specific inhibitors of DP-IV, with Kᵢ values in the nanomolar range.

Dipeptides having the boroPro moiety are relatively unstable; thus, as one possibility, we have designed inhibitors having at least two other amino acid residues. Generally, the structure of these inhibitors is X-Pro-Y-boroPro where X and Y are chosen from any amino acid residue (including proline). This tetrapeptide may be lengthened at its amino-terminus by addition of one or more dipeptides, each dipeptide having the general formula Z-Pro or Z-ala, where each Z independently is any amino acid residue (including proline). This general structure is defined in more detail below. These inhibitors function as inhibitors of DP-IV because each dipeptide portion is a substrate for DP-IV and the final product of the reaction of such an inhibitor with DP-IV is the dipeptide inhibitor Y-boroPro. The amino terminus of these inhibitors must not be blocked or they lose their inhibitory capacity for DP-IV, since DP-IV cannot cleave a dipeptide from a blocked N-terminal polypeptide.

In accordance with a first aspect of the invention, a purified inhibitor of the amino peptidase activity of dipeptidyl peptidase type IV (DP-IV) is provided, having the structure: 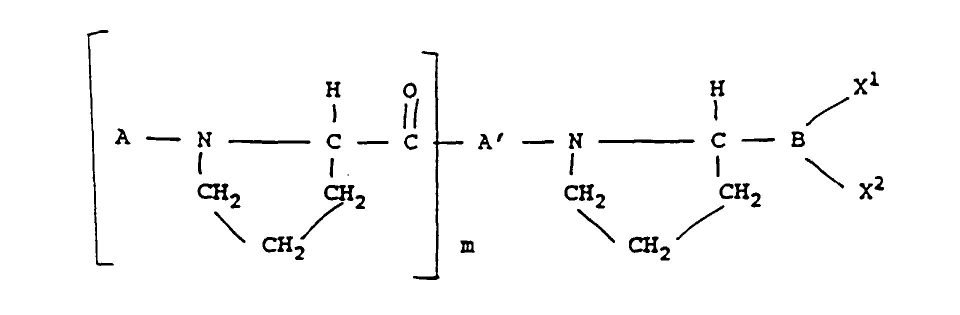 wherein m is an integer between 0 and 10, inclusive; A and A' are L-amino acid residues (for glycine there is no such distinction) such that the A in each repeating bracketed unit can be a different amino acid residue; the C bonded to B is in the L-configuration; the bonds between A and N, A' and C, and between A' and N are peptide bonds; and each X¹ and X² is, independently, a hydroxyl group or a group capable of being hydrolysed to a hydroxyl group at physiological pH. By "the C bonded to B is in the L-configuration" is meant that the absolute configuration of the C is like that of an L-amino acid. Thus the 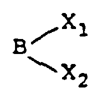 group has the same relationship to the C as the -COOH group of an L-amino acid has to its α carbon. In various preferred embodiments, A and A' are independently proline or alanine residues; m is 0; X¹ and X² are hydroxyl groups; the inhibitor is L-Ala-L-boroPro; and the inhibitor is L-Pro-L-boroPro.

Preferably, X¹ and X² from the general formula are hydroxyl groups.

In a further preferred embodiment, A and A' are independently proline or alanine residues.

In an inhibitor according to the present invention wherein m is 0, A' is desirably other than alanine.

One example of a preferred inhibitor according to the present invention is L-Ala-L-boroPro.

Another example of a preferred inhibitor according to the present invention is L-Pro-L-boroPro.

An inhibitor according desirably has an isomeric purity of about 96-99%.

According to a preferred embodiment, an inhibitor according to the first aspect of the invention is for use in inhibiting the enzymatic activity of DP-IV in a mammal, preferably for regulation of an immune response.

To that end, according to a second aspect of the invention, a pharmaceutical composition is provided comprising one or more inhibitors according to the first aspect of the invention, together with a pharmaceutical carrier or diluent.

According to a third aspect of the invention, a preparation is provided comprising an inhibitor according to the first aspect of the invention, or a composition according to the second aspect, wherein said inhibitor is present with an isomeric purity of about 96-99%.

A fourth aspect of the present invention provides the use of an inhibitor according to the first aspect of the invention, for the manufacture of a medicament for use in inhibiting the enzymatic activity of DP-IV in a mammal, preferably for regulation of an immune response.

In a preferred embodiment, the medicament is suitable for administering a dosage of 1 mg/kg to 500 mg/kg of the mammal per day.

Other features and advantages of the invention will be apparent from the following description of the preferred embodiments, and from the claims.

### Description of the Preferred Embodiments

The drawing will first be briefly described.

### Drawing

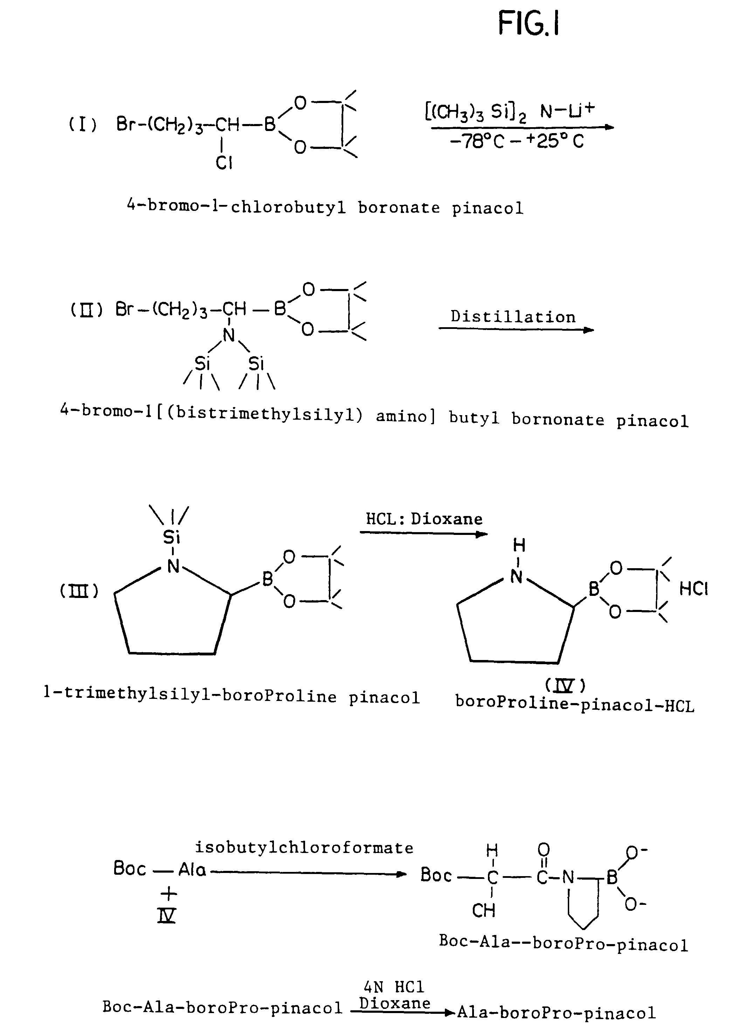

Fig. 1 is a diagrammatic representation of the synthesis of a boro proline compound.

### Structure

The inhibitory compounds of the invention have the general structure recited in the Summary of the Invention above. Examples of preferred structures are those referred to as preferred embodiments above.

The structure of the preferred inhibitory compounds is such that at least a portion of the amino acid sequence near the cleavage site of a DP-IV substrate is duplicated, or nearly duplicated. This duplication is in part responsible for the ability of the inhibitory compounds to inhibit DP-IV, by a mechanism thought to involve competitive inhibition between a DP-IV inhibitory compound or a DP-IV cleavage product of the inhibitory compound, and the actual DP-IV substrate.

The choice of amino acid sequence affects the inhibitory activity of the inhibitory compound, and its specificity. Peptide fragments can be synthesized and then tested to determine their efficacy as inhibitors, using standard techniques. Specificity is determined in a similar fashion, by testing the inhibitory effect of a particular inhibitory compound on the enzyme activity. The inhibitory compounds preferably inhibit the enzymatic activity of DP-IV and do not inhibit enzymes necessary for normal cell functions.

The inhibitory compounds include the group: 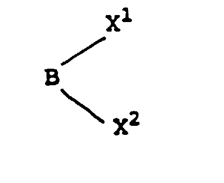 which causes the inhibitory compound to complex with DP-IV, not only in a competitive fashion, but in a chemically reactive manner to form a strong bond between the inhibitory compound and DP-IV. This group thus acts to bind the inhibitory compound to DP-IV, and increases the inhibitory binding constant (Kᵢ) of the inhibitory compound.

### Synthesis of BoroProline

Referring to Fig. 1, the starting compound I is prepared essentially by the procedure of Matteson et al. (*Organometallics* 3:1284, 1984), except that a pinacol ester is substituted for the pinanediol ester. Similar compounds such as boropipecolic acid and 2-azetodine boronic acid can be prepared by making the appropriate selection of starting material to yield the pentyl and propyl analogs of compound I. Further, Cl can be substituted for Br in the formula, and other diol protecting groups can be substituted for pinacol in the formula, e.g., 2, 3-butanediol and alpha-pinanediol.

Compound II is prepared by reacting compound I with [(CH₃)O₃Si]₂N-Li+. In this reaction hexamethyldisilazane is dissolved in tetrahydrofuran and an equivalent of n-butyllithium added at -78°C. After warming to room temperature (20°C) and cooling to -78°C, an equivalent of compound I is added in tetrahydrofuran. The mixture is allowed to slowly come to room temperature and to stir overnight. The alpha-bis[trimethylsilane]-protected amine is isolated by evaporating solvent and adding hexane under anhydrous conditions. Insoluble residue is removed by filtration under a nitrogen blanket, yielding a hexane solution of compound II.

Compound III, the N-trimethysilyl protected form of boroProline is obtained by the thermal cyclization of compound II during the distillation process in which compound II is heated to 100-150° and distillate is collected which boils 66-62°C at 0.06-0.10 mm pressure.

Compound IV, boroProline-pinacol hydrogen chloride, is obtained by treatment of compound III with HCl:dioxane. Excess HCl and by-products are removed by trituration with ether. The final product is obtained in a high degree of purity by recrystallization from ethyl acetate.

The boroProline esters can also be obtained by treatment of the reaction mixture obtained in the preparation of compound II with anhydrous acid to yield 1-amino-4-bromobutyl boronate pinacol as a salt. Cyclization occurs after neutralizing the salt with base and heating the reaction.

### Preparation of boroProline-pinacol

The intermediate, 4-Bromo-l-chlorobutyl boronate pinacol, was prepared by the method in Matteson et al. (*Organometallics* 3:1284, 1984) except that conditions were modified for large scale preparations and pinacol was substituted for the pinanediol protecting group.

3-bromopropyl boronate pinacol was prepared by hydrogenboronation of allyl bromide (173 ml, 2.00 moles) with catechol borane (240 ml, 2.00 moles). Catechol borane was added to allyl bromide and the reaction heated for 4 hours at 100°C under a nitrogen atmosphere. The product, 3-bromopropyl boronate catechol (bp 95-102°C, 0.25 mm), was isolated in a yield of 49% by distillation. The catechol ester (124 g, 0.52 moles) was transesterified with pinacol (61.5 g, 0.52 moles) by mixing the component in 50 ml of THF and allowing them to stir for 0.5 hours at 0°C and 0.5 hours at room temperature. Solvent was removed by evaporation and 250 ml of hexane added. Catechol was removed as a crystalline solid. Quantitative removal was achieved by successive dilution to 500 ml and to 1000 ml with hexane and removing crystals at each dilution. Hexane was evaporated and the product distilled to yield 177 g (bp 60 - 64°C, 0.35 mm).

4-Bromo-1-chlorobutyl boronate pinacol was prepared by homologation of the corresponding propyl boronate. Methylene chloride (50.54 ml, 0.713 moles) was dissolved in 500 ml of THF, 1.54 N n-butyllithium in hexane (480 ml, 0.780 moles) was slowly added at -100°C. 3-Bromopropyl boronate pinacol (178 g, 0.713 moles) was dissolved in 500 ml of THG, cooled to the freezing point of the solution, and added to the reaction mixture. Zinc chloride (54.4 g, 0.392 moles) was dissolved in 250 ml of THG, cooled to 0°C, and added to the reaction mixture in several portions. The reaction was allowed to slowly warm to room temperature and to stir overnight. Solvent was evaporated and the residue dissolved in hexane (1 liter) and washed with water (1 liter). Insoluble material was discarded. After drying over anhydrous magnesium sulfate and filtering, solvent was evaporated. The product was distilled to yield 147 g (bp 110 - 112°C, 0.200 mm).

N-Trimethylsilyl-boroProline pinacol was prepared first by dissolving hexamethyldisilizane (20.0 g, 80.0 mmoles) in 30 ml of THF, cooling the solution to -78°C, and adding 1.62 N n-butyllithium in hexane (49.4 ml, 80.0 mmoles). The solution was allowed to slowly warm to room temperature. It was recooled to -78°C and 4-bromo-1-chlorobutyl boronate pinacol (23.9 g, 80.0 mmoles) added in 20 ml of THF. The mixture was allowed to slowly warm to room temperature and to stir overnight. Solvent was removed by evaporation and dry hexane (400 ml) added to yield a precipitant which was removed by filbration under a nitrogen atmosphere. The filtrate was evaporated and the residue distilled, yielding 19.4 g of the desired product (bp 60 - 62°C, 0.1 - 0.06 mm).

H-boroProline-pinacol.HCl (boroProline-pinacol.HCl) was prepared by cooling N-trimethylsilyl-boroProline pinacol (16.0 g, 61.7 mmoles) to -78°C and adding 4 N HCL:dioxane 46 ml, 185 mmoles). The mixture was stirred 30 minutes at -78°C and 1 hour at room temperature. Solvent was evaporated and the residue triturated with ether to yield a solid. The crude product was dissolved in chloroform and insoluble material removed by filtration. The solution was evaporated and the product crystallized from ethyl acetate to yield 11.1 g of the desired product (mp 156.5 - 157°C).

### Synthesis of boroProline Peptides

General methods of coupling of N-protected peptides and amino acids with suitable side-chain protecting groups to H-boroProline-pinacol are applicable. When needed, side-chain protecting and N-terminal protecting groups can be removed by treatment with anhydrous HCl, HBr, trifluoroacetic acid, or by catalytic hydrogenation. These procedures are known to those skilled in the art of peptide synthesis.

The mixed anhydride procedure of Anderson et al. (*J. Am. Chem. Soc.* 89:5012, 1984) is preferred for peptide coupling. Referring again to Fig. 1, the mixed anhydride of an N-protected amino acid or a peptide is prepared by dissolving the peptide in tetrahydrofuran and adding one equivalent of N-methylmorpholine. The solution is cooled to -20°C and an equivalent of isobutyl chloroformate is added. After 5 minutes, this mixture and one equivalent of triethylamine (or other sterically hindered base) are added to a solution of H-boroPro-pinacol dissolved in either cold chloroform of tetrahydrofuran.

The reaction mixture is routinely stirred for one hour at -20°C and 1 to 2 hours at room temperature (20°C). Solvent is removed by evaporation, and the residue is dissolved in ethyl acetate. The organic solution is washed with 0.20N hydrochloric acid, 5% aqueous sodium bicarbonate, and saturated aqueous sodium chloride. The organic phase is dried over anhydrous sodium sulfate, filtered, and evaporated. Products are purified by either silica gel chromatography or gel permeation chromatography using sephadex™ LH-20 and methanol as a solvent.

Previous studies have shown that the pinacol protecting group can be removed *in situ* by preincubation in phosphate buffer prior to running biological experiments (Kettner et al., *J. Biol. Chem.* 259:15106, 1984). Several other methods are also applicable for removing pinacol groups from peptides, including boroProline, and characterizing the final product. First, the peptide can be treated with diethanolamine to yield the corresponding diethanolamine boronic acid ester, which can be readily hydrolyzed by treatment with aqueous acid or a sulfonic acid substituted polystyrene resin as described in Kettner et al. (*supra*). Both pinacol and pinanediol protecting groups can be removed by treating with BC13 in methylene chloride as described by Kinder et al. (*J. Med. Chem.* 28:1917). Finally, the free boronic acid can be converted to the difluoroboron derivative (-BF2) by treatment with aqueous HF as described by Kinder et al. (*supra*).

Similarly, different ester groups can be introduced by reacting the free boronic acid with various di-hydroxy compounds (for example, those containing heteroatoms such as S or N) in an inert solvent.

### Preparation of H-Ala-boroPro

Boc-Ala-boroPro was prepared by mixed anhydride coupling of the N-Boc-protected alanine and H-boroPro prepared as described above. H-Ala-boroPro (Ala-boroPro) was prepared by removal of the Boc protecting group at 0°C in 3.5 molar excess of 4N HCl-dioxane. The coupling and deblocking reactions were performed by standard chemical reaction. Ala-boroPro has a Kᵢ for DP-IV of in the nanomolar range. Boc-blocked Ala-boroPro has no affinity for DP-IV.

One significant drawback with H-Ala-boroPro as an inhibitor for DP-IV is that is decomposes in aqueous solution at neutral pH and room temperature (20 - 25°C) with a half-life of around 0.5 hour. Many dipeptide derivatives with a free N terminal amino group and a functional group (such as a difluoromethyl ketone) on the C-terminus are similarly unstable due to intramolecular reaction. A six member ring is formed between the amino and C-terminal functional groups and undergoes subsequent further reaction, such as hydrolysis. DP-IV bound inhibitor is more stable, consistent with the hypothesis that decomposition is due to an intramolecular reaction.

H-Pro-boroPro is more stable than H-Ala-boroPro. The Kᵢ of H-Pro-boroPro for DP-IV is about 1 x 10⁻⁸M, and it decomposes in aqueous solution at room temperature (20-25°C) with a half life of about 1.5 hours. Although the affinity of H-Pro-boroPro is about 10-fold less than that of H-Ala-boroPro, the increased stability is advantageous.

Because of the relatively short half life of the above dipeptides our inhibitory compounds are formed as tetrapeptides or longer peptides in one aspect of the invention. These inhibitory compounds are substrates for DP-IV yielding the dipeptide inhibitor A'-boroPro. These polypeptide boronic acids are generally stable and can be administered by any standard procedure to act as a substrate for DP-IV and then as a source of a potent DP-IV inhibitor. The advantages of such molecules is that inhibitor is released only in the vicinity of active DP-IV. These polypeptide boronic acids can be made by the method of mixed anhydride coupling by one of ordinary skill in the art, e.g., Mattason *(Organometallics* 3:1284, 1984).

### Assays for DP-IV Inhibition

The following are examples of systems by which the inhibitory activity of the above described inhibitory compounds can be tested on DP-IV. As an example H-Ala-boroPro though not a compound in accordance with the present invention is described as being used to test each of these systems.
Inhibitory compounds can be tested by simply substituting them for H-Ala-boroPro.

DP-IV is purified from pig kidney cortex by the method of Barth et al. (*Acta Biol. Med. Germ.* 32:157, 1974) and Wolf et al. (*Acta Biol. Med. Germ.* 37:409, 1978) and from human placenta by the method of Puschel et al. (*Eur. J. Biochem.* 126:359, 1982). H-Ala-boroPro inhibits both enzymes with a Kᵢ in the nanomolar range.

### Human Peripheral blood Mononuclear Cells

H-Ala-boroPro was tested for its influence on PHA-induced proliferation of human peripheral blood mononuclear cells. Human peripheral blood mononuclear cells were obtained from healthy human donors by Ficoll-Hypaque density gradient centrifugation. The cells are washed three times in RPMI 1640 medium and resuspended to a concentration of a 1 x 10⁶ in RPMI. 10% human serum was used as necessary.

The proliferative response of lymphocytes was measured using 3H-Thymidine incorporation. 5 x 10³ MNC cells (Ford in *Handbook of Experimental Immunology,* Weir, ed., Blackwell Scientific Publications, Oxford, 1978) were distributed into wells of round-bottom microtiter plates and incubated in the presence or absence of various dilutions of antigen, mitogen, lymphokine or other agent of interest. Cells were cultured in an atmosphere of 5% CO₂ in air for 72 hours after which ³H-Thymidine (0.5 µC1/well; 2.0 Ci/mM, New England Nuclear) was added 6 hours before termination of culture. The cells were harvested with a multiple automatic harvester, and ³H-thymidine incorporation assessed by liquid scintillation counting. ³H thymidine incorporation was determined relative to control values in the absence of inhibitor. Inhibitor was added to give a final concentration of 1 x 10⁻⁴M, but lower concentrations can be used.

### HIV gene replication

We examined the effect of H-Ala-boroPro on HIV-1 replication *in vitro.* The rational for these experiments comes from the reported connection between T-cell activation, IL-2 production, and HIV replication and expression of HIV proteins. For example, inductive signals associated with HIV replication include mitogens, antigens, lymphokines, and transcriptions factors such as NF-κB, all of which have been shown to be associated with induction of IL-2 production, T-cell activation, or both.

Cell lines used in the present studies include A3.5 cells (a monocyte cell line which is CD4+, HLA-DR+, and CD3-) and peripheral blood mononuclear cells (PBMC). The A3.5 cells grow continuously in culture without exogenous growth factors. PBMC cells require IL-2 for propagation *in vitro.* Cells were infected with HIV-1-IIIB at a multiplicity of infection (moi) of 5 x 10⁻⁴ tissue culture infectious dose 50 (TCID50)/cell for both the A3.5 cells and the PMBC cells. Dilutions of inhibitor were made in RPMI-1640 and subsequently passed through a 0.22 um filter. At the start of each experiment, 1 x 10⁶ cells/well, in 24-well plates, were infected with HIV-1-IIIB at the moi indicated above. Inhibitor was added simultaneously at the appropriate dilutions. All cultures were maintained at 5% CO₂ and 37°C in RPMI-1640 supplemented with penicillin, streptomycin, L-glutamine, hepes buffer, and 20% heat-inactivated fetal calf serum. Cell counts and viability were determined by trypan blue exclusion. Culture supernatants were harvested and assayed for HIV-1 p24 antigen by ELISA (NEN-DuPont, Boston, MA). Fresh media and inhibitor were added on each day. For PBMC cultures, cells were collected from HIV-1 seronegative donors and stimulated with PHA-P (Difco, Detroit, MI; 10 µg/ml) and 10% IL-2 (Electronnucleonics, Silver Spring, MD) 3 days prior to infection with HIV-1. PBMC cultures for all experiments included uninfected and infected cells without inhibitor, uninfected cells with inhibitor at the various concentrations, and infected cells in the presence of 1 µm zidovudine (azidothymidine, AZT).

With A3.5 H-Ala-boroPro suppresses HIV below detectable levels in a manner similar to the anti-HIV effect of AZT at 1 µm. Similar results were observed with the PBMC cells. Thus, the inhibitors have an anti-HIV effect. Cell viability assays show that these inhibitors are not cytotoxic even at relatively high concentration (10⁻³M for A3.5 cells).

### Determination of DP-IV Activities in Biological samples

The ability to determine DP-IV activities associated with cells and tissues is highly desirable. For example, it will permit correlations to be made between level of inhibition of DP-IV and the magnitude of the observed biological affect, e.g., on cell proliferation, and IL-2 production. Such correlation is helpful in establishing whether or not the biological effect is due to inhibition of DP-IV. We have found that such determinations can be reproducibly and reliably made using the readily available chromogenic substrates for DP-IV: X-Pro-p-nitroanilides and X-Pro-7-amino-4-trifluoromethyl coumarins (AFC). The AFC substrates are fluorescent and thus provide greater sensitivity. DP-IV activity is measured as release of p-nitroanilide spectrophotometrically at 410 nM, or using X-Pro-AFC derivatives and measuring fluorescence at 505 nM. Reduction in activity in the presence of inhibitor provides an easy test for inhibitory activity.

### Effect of Inhibitor Stereochemistry on DP-IV Inhibition

Experiments described below demonstrate that Ala-boroPro and Pro-boroPro are potent inhibitors of DP-IV with Kᵢ values in the nanomolar range. In addition, the L,L form of Pro-boroPro is shown to be a far more potent inhibitor of DP-IV than the L,D form of Pro-boroPro.

The activity of DP-IV, isolated from porcine kidneys by the method of Wolf et al. (*ACTA Bio. Mes. Ger.* 37:409, 1972), was measured using Ala-Pro-p-nitroanilide as a substrate. Briefly, a reaction containing 50 µmol sodium Hepes (pH7.8), 10 µmol Ala-Pro-*p*-nitroanilide, 6 milliunits of DP-IV, and 2% (vol/vol) dimethylformamide in a total volume of 1.0 ml. The reaction was initiated by the addition of enzyme and reaction rates were measured at 25°C.

The rates of DP-IV-catalyzed hydrolysis of Ala-Pro-p-nitroanilide were determined at 3 to 5 different concentration of Ala-boroPro Pro-boroPro, boroPro and *N*-Boc-Ala-boroPro. In some cases, the initial reactions rates were not linear. The rates became linear after 10 min; this linear portion can be duplicated by preincubating enzyme with inhibitor for 10 min before adding substrate. Table 1 presents the results of Kᵢ measurements made over the linear range.

**Table 1:**

| Inhibition constants of some inhibitors of DP-IV | |
|---|---|
| Inhibitor | Kᵢ nM |
| N-Boc-Al-boroPro | >1,000,000* |
| BoroPro | 110,000 |
| Ala-boroPro | 2 |
| Pro-boroPro | 3 |

| | |
|---|---|
| *No inhibition detected. | |

Ala-boroPro was a potent inhibitor of DP-IV, having a Kᵢ value of 2 x 10⁻⁹M (Table 1). Blocking the N terminus of this inhibitor (e.g., *N*-Boc-Ala-boroPro; Table 1) abolished inhibition, demonstrating that a free, positively charged amino group is likely essential for enzyme recognition and binding. The Kᵢ of 3 x 10⁻⁹ M for Pro-boroPro demonstrates that DP-IV tolerates an imino group in place of the amino functional group on the N terminus as well as the substitution of a proline side chain in place of the alanine methyl group. This shows that the S2 specificity subsite is not highly restrictive. Although DP-IV will accept nearly any amino acid at the N terminus, interactions between this amino acid and the enzyme are critical for binding. This is illustrated by the 10⁵-10⁶ decrease in affinity on going from Ala-boroPro or Pro-boroPro to boroPro itself (Table 1).

The inhibition experiments presented in Table 1 were carried out on DP-IV isolated from pig kidneys. Pro-boroPro and Ala-boroPro inhibit DP-IV from human placenta equally well.

The Ala-boroPro and Pro-boroPro used in the experiments described above were raecemic mixtures in which the boroPro moiety was present as both the D-form and L-form while Ala and Pro were both the L-isomer.

High pressure liquid chromatography (HPLC) can be used to separate L-Pro-D-boroPro from L-Pro-L-boroPro. A 4.6 mm x 250 mm Nucleosil C18 (5µ particle) column employing a two buffer system (Buffer A is 100% H₂O with 0.1% TFA, and buffer B is 70% CH₃CN, 30% H₂O, 0.86% TFA) can be used to carry out the seperation. From 0 to 5 min 5% B and 95% A is used, and from 5 to 25 min 5% to 100% B is used. The L,L isomer comes off first at about 7 min, followed by the L,D isomer at about 10 min. NMR and mass spectra analysis were consistent with both compounds being Pro-boroPro. Rechromatography of the purified isomers indicated that the first pass on the HPLC column achieved an isomeric purity of about 99-6% for each isomer. High pressure liquid chromatography (HPLC) can similarly be used to be used to separate L-Ala-D-boroPro from L-Ala-L-boroPro or to seperate the D-boroPro form of other inhibitors from the L-boroPro form.

When L-Pro-L-boroPro and L-Pro-D-boroPro were used in a DP-IV inhibition assay, the Kᵢ for L-Pro-L-boroPro was 3.2 x 10⁻¹¹M, while for L-Pro-D-boroPro the Kᵢ was 6.0 x 10⁻⁸M. The L,L-isomer constitutes a much better inhibitor for DP-IV than the L,D-isomer. Further it is preferred that all of the amino acid residues of the DPIV inhibitors of the invention be the L-isomer rather than the D-isomer.

The inhibitory compounds can be administered in an effective amount either alone or in combination with a pharmaceutically acceptable carrier or diluent.

The above inhibitory compounds are useful for treatment of a wide variety of disease; for example, an autoimmune disease, the pathogenesis of which is dependent on T cell activity. DP-IV plays a role in such autoimmune disease and inhibition of DP-IV activity allows regulation of the progress of the disease. Such diseases include arthritis, rejection of transplanted organs, as well as SLE and AIDS. When administered to mammals (e.g., orally, topically, intramuscularly, intraperitoneally, intravenously, parenterally, nasally or by suppository), the inhibitory compounds of this invention enhance the ability of, e.g., the immune system of the mammal, to fight the disease.

Inhibitors of DP-IV can suppress IL-2 production and thus diseases in which the production of IL-2 is altered may be treated by use of these inhibitors. These inhibitors can also delay catabolism of growth hormone releasing factor, and block DP-IV activity of amoebae and microbial pathogens to allow an immune system to act more efficiently.

The inhibitory compounds or compositions can be administered alone or in combination with one another, or in combination with other therapeutic agents. The dosage level may be between 1 - 500 mg/kg/day.

Other embodiments are feasible. For example, other L,L-isomer inhibitors can be created which mimic the structure of Ala-boroPro.

## Claims

1. A purified inhibitor of the amino peptidase activity of dipeptidyl peptidase type IV (hereafter "DP-IV"), having the structure: 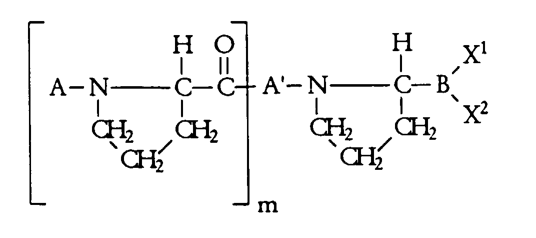 wherein m is an integer between 0 and 10, inclusive; A and A' are L-amino acid residues such that the A in each repeating bracketed unit can be a different amino acid residue; the C bonded to B is in the L-configuration; the bonds between A and N, A' and C, and between A' and N are peptide bonds; and each X¹ and X² is, independently, a hydroxyl group or a group capable of being hydrolysed to a hydroxyl group at physiological pH.

2. An inhibitor as claimed in claim 1, wherein X¹ and X² are hydroxyl groups.

3. An inhibitor as claimed in claim 1 or claim 2, wherein A and A' are independently proline or alanine residues.

4. An inhibitor as claimed in any of claims 1-3, wherein m is 0.

5. An inhibitor as claimed in any of claims 1-4, with the proviso that, when m is 0, A' is other than Ala.

6. An inhibitor as claimed in any of claims 1-4, wherein said inhibitor is L-Ala-L-boroPro.

7. An inhibitor as claimed in any of claims 1-5, wherein said inhibitor is L-Pro-L-boroPro.

8. An inhibitor as claimed in any of claims 1-7, with an isomeric purity of about 96-99%.

9. A pharmaceutical composition comprising one or more inhibitors according to any of claims 1 to 8, together with a pharmaceutical carrier or diluent.

10. A preparation comprising an inhibitor as claimed in any of claims 1-8, or a composition as claimed in claim 9, wherein said inhibitor is present with an isomeric purity of about 96-99%.

11. An inhibitor as claimed in any of claims 1-8, for use in inhibiting the enzymatic activity of DP-IV in a mammal, preferably for regulation of an immune response.

12. Use of an inhibitor as claimed in any of claims 1-8, for the manufacture of a medicament for use in inhibiting the enzymatic activity of DP-IV in a mammal, preferably for regulation of an immune response.

## Patentansprüche

1. Gereinigter Inhibitor für die Aminopeptidase-Aktivität von Dipeptidylpeptidase Typ IV (im folgenden "DP-IV") mit der Struktur: 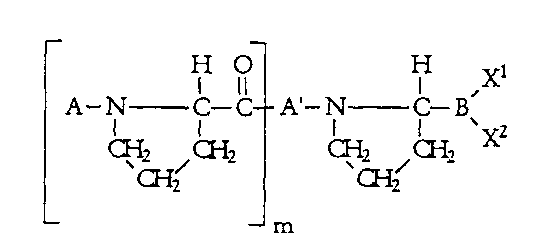 worin m eine ganze Zahl zwischen einschließlich 0 und 10 ist, A und A' L-Aminosäurereste sind, wobei das A in jeder eingeklammerten Struktureinheit ein anderer Aminosäurerest sein kann; das an B gebundene C in der L-Konfiguration vorliegt; die Bindungen zwischen A und N, zwischen A' und C und zwischen A' und N Peptidbindungen sind; und X¹ und X² jeweils unabhängig eine Hydroxylgruppe bedeuten oder eine Gruppe, die bei physiologischem pH zu einer Hydroxylgruppe hydrolysiert werden kann.

2. Inhibitor nach Anspruch 1, worin X¹ und X² Hydroxylgruppen sind.

3. Inhibitor nach Anspruch 1 oder Anspruch 2, worin A und A' unabhängig voneinander Prolin- oder Alaninreste bedeuten.

4. Inhibitor nach irgendeinem der Ansprüche 1-3, worin m 0 ist.

5. Inhibitor nach irgendeinem der Ansprüche 1-4, mit der Maßgabe, daß A' nicht Ala ist, wenn m 0 ist.

6. Inhibitor nach irgendeinem der Ansprüche 1-4, worin der Inhibitor L-Ala-L-boroPro ist.

7. Inhibitor nach irgendeinem der Ansprüche 1-5, worin der Inhibitor L-Pro-L-boroPro ist.

8. Inhibitor nach irgendeinem der Ansprüche 1-7 mit einer Isomerenreinheit von ungefähr 96-99%.

9. Pharmazeutische Zusammensetzung umfassend einen oder mehrere Inhibitoren nach irgendeinem der Ansprüche 1-8 zusammen mit einem pharmazeutischen Träger oder Verdünnungsmittel.

10. Präparat umfassend einen Inhibitor nach irgendeinem der Ansprüche 1-8 oder eine Zusammensetzung nach Anspruch 9, worin der Inhibitor in einer Isomerenreinheit von ungefähr 96-99% vorliegt.

11. Inhibitor nach irgendeinem der Ansprüche 1-8 zur Inhibierung der enzymatischen Aktivität von DP-IV in einem Säuger, vorzugsweise zur Steuerung einer Immunantwort.

12. Verwendung eines Inhibitors nach irgendeinem der Ansprüche 1-8 zur Herstellung eines Medikaments zur Inhibierung der enzymatischen Aktivität von DP-IV in einem Säuger, vorzugsweise zur Steuerung einer Immunantwort.

## Revendications

1. Un inhibiteur purifié de l'activité amino-peptidase de la dipeptidyl peptidase de type IV (désignée ci-après par « DP-IV »), ayant la structure : 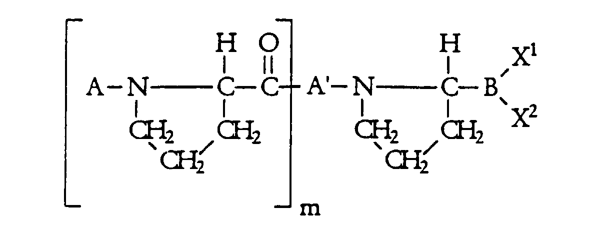 dans laquelle m est un nombre entier compris entre 0 et 10 inclusivement ; A et A' sont des restes L-aminoacide tel que le symbole A dans chacune des unités répétitives entre crochets peuvent représenter des restes aminoacides différents ; le C relié à B est en configuration L, les liaisons entre A et N, A' et C et entre A' et N sont des liaisons peptidiques et chacun des symboles X¹ et X² est indépendamment un groupe hydroxyle ou un groupe susceptible d'être hydrolysé en un groupe hydroxyle au pH physiologique.

2. Un inhibiteur selon la revendication 1, dans lequel X¹ et X² sont des groupes hydroxyles.

3. Un inhibiteur selon la revendication 1 ou la revendication 2, dans lequel A et A' sont indépendamment des restes proline ou alanine.

4. Un inhibiteur selon l'une quelconque des revendications 1-3, dans lequel m est égal à 0.

5. Un inhibiteur selon l'une quelconque des revendications 1-4, dans lequel quand m est égal à 0, A' est différent de Ala.

6. Un inhibiteur selon l'une quelconque des revendications 1-4, dans lequel l'inhibiteur est la L-Ala-L-boroPro.

7. Un inhibiteur selon l'une quelconque des revendications 1-5, dans lequel ledit inhibiteur est la L-Pro-L-boroPro.

8. Un inhibiteur selon l'une quelconque des revendications 1-7, dont la pureté isomère est d'environ 96-99 %.

9. Une composition pharmaceutique comprenant un ou plusieurs inhibiteurs selon l'une quelconque des revendications 1 à 8, en même temps qu'un véhicule ou diluant pharmaceutique.

10. Une préparation comprenant un inhibiteur selon l'une quelconque des revendications 1-8 ou une composition selon la revendication 9, dans laquelle ledit inhibiteur est présent avec une pureté isomère d'environ 96-99 %.

11. Un inhibiteur selon l'une quelconque des revendications 1-8, destiné à être utilisé pour inhiber l'activité enzymatique de la DP-IV chez un mammifère, de préférence en vue de la régulation d'une réponse immunitaire.

12. Application d'un inhibiteur selon l'une quelconque des revendications 1-8, destiné à la préparation d'un médicament utilisable pour inhiber l'activité enzymatique de la DP-IV chez un mammifère, de préférence pour la régulation d'une réponse immunitaire.
